# EUROPEAN PATENT APPLICATION

(11) **EP 1 757 317 A1**
(43) Date of publication of application: **28.02.2007**
(21) Application number: 05255250.2
(22) Date of filing: 25.08.2005
(51) Int. Cl.: A61M 1/00, F04F 5/52

(54) **Compensating venturi vacuum system**

(71) Applicant: Ohio Medical Corporation, Gurnee IL 60031 (US)
(72) Inventor: Orwig, Steven J., Bowie, MD 20720 (US)
(74) Representative: Hedley, Nicholas James Matthew

(57) **Abstract**

A venturi-based vacuum system that provides a compensated vacuum supply to a vacuum utilization device. The system creates a vacuum by means of a source gas (32) passing through a standard venturi (24) with a flow control valve (34) controlling the flow of that source gas and thus, the level of vacuum produced. A feedback system is provided that makes the vacuum system self-compensating. The feedback system senses the level of vacuum in a vacuum supply outlet (38) to the vacuum utilization device (40) and uses that sensed level to control the setting of the flow control valve (34). As such, changes in flow in the vacuum utilization device that result in changes in the vacuum level from the vacuum system are compensated for by a corresponding change in the setting of the flow control valve to maintain the vacuum level at a constant level.

## Description

### Background of the Invention

The present invention relates to a vacuum system and, more particularly, to a vacuum system that provides a regulated supply of vacuum by means of a venturi system.

During the care of patients in a medical care institution, it is normal to use vacuum to carry out certain procedures on a patient, for example, to suck fluids from an internal cavity of patient or in the removal of fluids from an incision or simply for purpose of resuscitation. In any event, there is a need for a source of vacuum to be available to a caregiver at the location of the patient.

In many hospitals, it is common to have a supply of vacuum that is provided by a centrally located vacuum pumping system and the vacuum created is then distributed to the various rooms within the hospital by means of a vacuum pipeline system so that the caregiver only needs to plug the particular piece of vacuum operated equipment into a pipeline outlet accessible in the room.

As another source of vacuum that is actually generated in an individual hospital room, there is commonly used a venturi-type of system that produces a vacuum through a venturi where compressed gas, such as air, also available in hospital rooms, is passed through a venturi to create a vacuum at or proximate to the throat of the venturi. As such, rather than requiring a central vacuum pump and the associated piping to supply that vacuum to an individual location within a hospital, the use of a venturi-type vacuum system creates the source of vacuum on a localized level for the particular use of that vacuum.

One of the difficulties with the venturi-type of vacuum system, however, is that the system cannot compensate for changes in the flow rate of the output vacuum provided that results in a change in the vacuum level used by the particular piece of apparatus, that is, if the vacuum level changes due to a change in flow of the vacuum used, the normal system is unable to compensate for itself to maintain the level of vacuum at a constant level. And, of course, the vacuum flow is limited by the flow of the source compressed air that passes through the venturi.

It would be therefore advantageous to have a vacuum system that generates a supply of vacuum by means of a venturi that is self regulating, that is, if the level of vacuum supplied by the venturi system changes, for example, due to some change in the flow of the vacuum in a vacuum supply line, there is a means to adjust the level of the vacuum back to its original level so the despite changes in the demand for vacuum and change in flow in the vacuum supply line, the vacuum level created by the system will remain constant.

### Summary of the Invention

The present invention relates to a vacuum system wherein the vacuum is created by means of a venturi that is formed within a venturi manifold. Accordingly, the venturi manifold has a source gas inlet where the flow of pressurized gas can enter the venturi manifold to pass through the venturi. In a normal hospital setting there would be a source of pressurized air that can be used as the source gas and which is available at various locations throughout the hospital.

There is also a bleed gas outlet in the venturi manifold that allows the source gas to leave the venturi manifold after passing through the venturi. As is known, therefore, the flow of the source gas through the venturi creates a vacuum at or proximate to the throat of the venturi and therefore there is also a vacuum supply outlet so that some conduit or other means can be connected to the venturi manifold in order to communicate that vacuum to some utilization device. Again, in the hospital setting, that device can be a resuscitator, drainage apparatus or some other suction device that removes fluids from the patient.

A flow control valve is used and which is located upstream of the source gas inlet so that the flow of the source gas to the venturi can be controlled. In accordance with the present invention, there is a feedback system that can vary the setting of the flow control valve in order to change the flow of source gas through the venturi. Thus, a vacuum sensor is located that can ascertain the vacuum indicative of the vacuum at the vacuum supply outlet. By that is meant, the sensor can be located at the vacuum supply outlet itself or can be located further upstream within a vacuum conduit providing the vacuum to a vacuum utilization device, or even at the vacuum utilization device itself, it only being of importance that there is some means of sensing the vacuum in a relative sense that is being created by the venturi.

With the present feedback system, therefore, the sensed vacuum is used to control the setting of the flow control valve in accordance with the vacuum level determined by the vacuum sensor. As such, for example, if there is an increase in the flow within the vacuum utilization device resulting in the absolute pressure increasing, that is, the vacuum level is being reduced, the feedback system senses that increase in absolute pressure and changes the setting of the flow control valve to increase the flow of source gas through the venturi hereby increasing the negative pressure at the vacuum supply outlet to maintain a constant level of vacuum supplied by the vacuum system.

As used herein, by convention, the level of vacuum will be referred to as increasing or decreasing. To eliminate confusion, when the level of vacuum is increasing, it is meant that the vacuum is getting stronger and the absolute pressure is being lowered, whereas a reduction in vacuum will mean that absolute pressure is increasing.

As can therefore be seen, there can be change in the flow in the vacuum conduit or vacuum utilization device of the venturi created vacuum, resulting in a change in the level of vacuum thereby supplied, and by means of the feedback system, the flow control valve can be opened or closed to increase or decrease the flow of source gas through the venturi to compensate for the change in vacuum level and thus maintain the vacuum level at a relative constant value.

In one embodiment, the feedback system can be comprised of a variable volume chamber where one or more walls of the chamber are movable and can be made of a diaphragm or flexible material. A conduit communicates from the interior of the variable volume chamber to a point in the vacuum system where the vacuum is being sensed. Thus, when the vacuum level decreases, that is, the absolute pressure becomes less negative, the variable volume chamber flexes inwardly and an actuator affixed to that chamber can operate the flow control valve to adjust the flow of source gas to the venturi to increase that flow to restore the vacuum level back to its original level.

Obviously, other schemes can be used for the feedback system. For example, the vacuum system can be an electronic sensor that sends an electrical signal to a flow control valve in accordance with the sensed vacuum and the flow control valve can be operated by means such as a stepper motor to change the valve setting responsive to the electrical signal. Other means of carrying out the feedback system can be by means of an optical vacuum sensor and optic fibers to carry those sensed signals to operate the flow control valve.

These and other features and advantages of the present invention will become more readily apparent during the following detailed description taken in conjunction with the drawings herein.

### Brief Description of the Drawings

FIG. 1 is a schematic view of a conventional venturi vacuum system; and
FIG. 2 is a schematic view of a venturi vacuum system constructed in accordance with the present invention.

### Detailed Description of the Invention

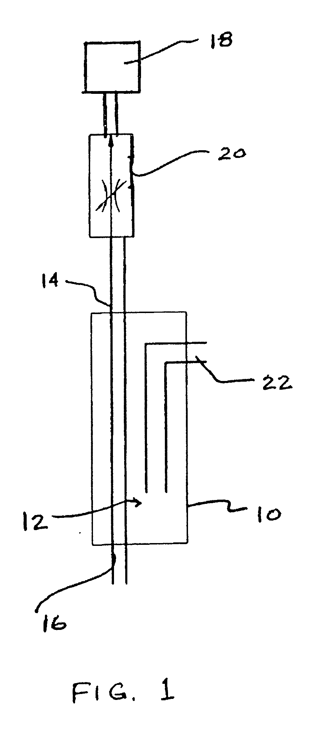

Referring now to Fig. 1, there is shown a schematic view of a conventional venturi system used to create a source of vacuum, for example, for a medical use in attending to a patient in a health care facility. As can be seen, there is a venturi manifold 10 and within which is formed a venturi 12. The venturi manifold 10 has a source gas inlet 14 and a bleed gas outlet 16. A gas source 18 is also shown and which can be any supply of a compressed gas that provides a flow of gas through the venturi 12. There is also a flow control valve 20 that is present upstream of the source gas inlet 14 in order to manually control the flow of the source gas through the venturi 12.

There is also a vacuum supply outlet 22 in the venturi manifold 10 where vacuum is available for an end use such as with a vacuum utilization device. As is well known, therefore, the flow of the source gas from the gas source 18, and controlled by the flow control valve 20, flows through the venturi 12 and is discharged outwardly of the venturi manifold 10 via the bleed gas outlet 16. That flow of gas creates a vacuum at or proximate to the throat of the venturi 10 and such vacuum is therefore available for an end use at the vacuum supply outlet.

With this conventional vacuum system, the flow of the source gas is established by the non-compensating flow control valve 20 which delivers a fixed flow rate of the source gas to the venturi 12. The relationship of the source gas flow rate V₁ is directly proportional to the maximum "static" vacuum generated by the venturi 12. Likewise, the maximum pressure of the source gas at the source gas inlet 14, P, is directly proportional to the maximum open flow (flow to atmosphere) that the vacuum system is capable of delivering. As the flow rate on the vacuum side, that is, upstream of the vacuum supply outlet 22 increases, the vacuum level within the system decreases rapidly depending on the environment. Depending upon the efficiency of the venturi system, the dynamic flow rate may be very low resulting in a more rapid decline in the corresponding dynamic vacuum level. When low maximum levels of vacuum are required, the corresponding flow is limited and which results in a greater reduction in the vacuum level at the vacuum supply outlet 22 in a dynamic environment.

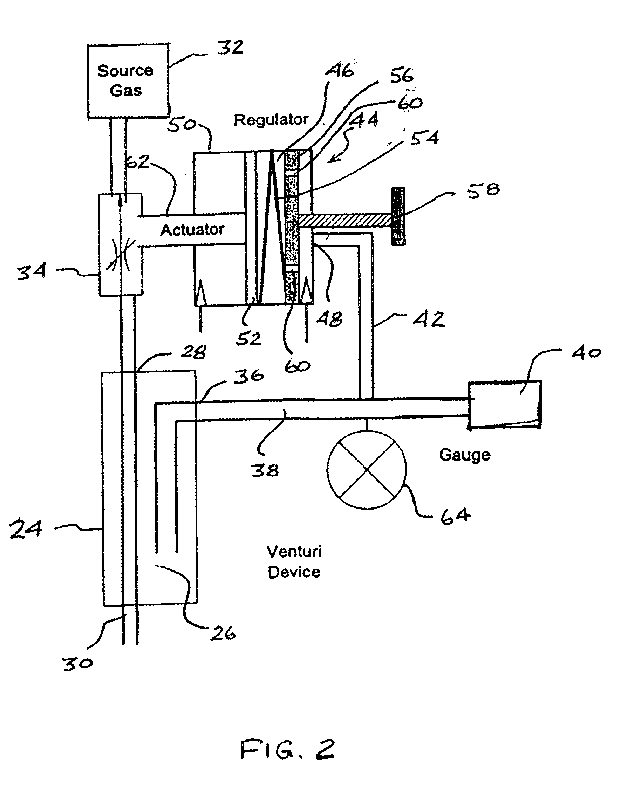

Turning now to Fig. 2, there is shown a schematic view of a venturi based vacuum system constructed in accordance with the present invention. In Fig. 2, there can be seen a venturi manifold 24 and within which is formed a venturi 26 similar to that described with respect to the Fig. 1 schematic. The venturi manifold 24 has a source gas inlet 28 and a bleed gas outlet 30. A gas source 32 is also shown and which, again, supplies the compressed gas to establish a flow of gas through the venturi 26. There is also a flow control valve 34 that is present upstream of the source gas inlet 28 in order to control the flow of the source gas through the venturi 26 in a manner to be described.

A vacuum supply outlet 36 is provided in the venturi manifold 24 where vacuum is available for an end use such as with a vacuum utilization device. As is the same as previously described, therefore, basically the flow of the source gas from the gas source 32 flows through the venturi 26 and is discharged outwardly of the venturi manifold 24 via the bleed gas outlet 30. That flow of gas creates a vacuum at or proximate to the throat of the venturi 26 and such vacuum is therefore available for an end use at the vacuum supply outlet 36.

With the Fig. 2 system, however, there is also a vacuum conduit 38 extending from the vacuum supply outlet 36 and which leads to some vacuum utilization device 40. As indicated, for a medical environment, the vacuum utilization device may be a resuscitator, suction apparatus or the like.

The vacuum feedback system of the present invention is illustrated in Fig. 2 where there is a feedback conduit 42 that communicates with the vacuum conduit 38 and which thereby transmits that level of vacuum to a regulator 44. The regulator 44 has a variable volume chamber 46 with an opening 48 in the variable volume chamber 46 for transmission of the level of vacuum in the vacuum conduit 38. The variable volume chamber 46 is formed by the exterior walls of a container 50 which has a movable wall 52 that moves depending upon the level of vacuum within the variable volume chamber 46. The movable wall 52 can be a diaphragm constructed of a flexible material.

A spring 54 can exert a pressure on the movable wall 52 and that pressure can be adjusted by manually moving an adjusting wall 56 that is controlled by a thumbscrew 58 to adjust the location of the adjusting wall 56 within the container 50 to allow a user to select the proper force exerted by the spring 54 against the movable wall 52. There are one or more apertures 60 provided in the adjusting wall 56 to allow the vacuum level in the feedback conduit 42 to communicate with the interior of the variable volume chamber 46.

An actuator 62 extends from the movable wall 52 to the flow control valve 34 so that the movement of the movable wall 52 is transmitted to the flow control valve 34 to change the setting of that flow control valve 34 in accordance with the position of the movable wall.

Accordingly, the operation of the feedback system can now be explained. As with the conventional vacuum system, the creation of the vacuum itself is accomplished as previously described by passing a flow of gas through the venturi 26. The vacuum is then present at the vacuum supply outlet 36 and transmitted to the vacuum utilization device 40 through the vacuum conduit 38. That level of vacuum is, in effect, sensed by the feedback conduit 42 where it is transmitted to the interior of the variable volume chamber 46 through the apertures 60 in the adjusting wall 56. A vacuum gauge 64 can also be employed in order to provide the user with a visual indication of the vacuum level in the vacuum conduit 42.

Thus, the level of vacuum in the vacuum conduit 38 will determine the location of the movable wall 52 since it can move laterally within the container 50 depending upon the level of vacuum in that variable volume chamber 46 due to a differential pressure across that movable wall 52, inasmuch as one side of the variable wall 52 is subjected to the vacuum level within the variable volume chamber 46 while the other side of the movable wall 52 is at ambient conditions. The movement of the movable wall 52 is, in turn, communicated to the flow control valve 34 where the movement is converted to a motion of the flow control valve 34 to change the setting of that valve establishing the flow of source gas through the venturi 26.

According as an example of the self-regulation of the present compensating vacuum system, if the vacuum utilization device requires an increased flow, the level of vacuum in the vacuum conduit 38 will be reduced, that is, the absolute pressure within the vacuum conduit will be raised. That raise in absolute pressure will be communicated to the variable volume chamber 46, resulting in the movable wall 52 moving to the left in Fig. 2 since the variable volume chamber 46 will expand due to the higher level of absolute pressure (lower level of vacuum or negative pressure).

That movement of the movable wall 52 acts through the actuator 62 to change the setting of the flow control valve 34 to further open that flow control valve 34 to increase the flow through the venturi 26 to raise the level of vacuum, that is, decrease the level of the absolute pressure in the vacuum conduit 38 so that the overall system is self-compensating such that the vacuum setting desired for the vacuum utilization device 40 remains constant despite changes in the flow therethrough and the vacuum level is maintained independent of the flow through the vacuum utilization device 40. For the same reasoning, therefore, the vacuum flow through the vacuum utilization device 40 is independent of the vacuum setting desired for the vacuum utilization device 40.

As previously stated, the vacuum sensor can be carried out by a variety of sensors, including an electronic vacuum sensor or fluidic vacuum sensor and the appropriate signal representative of the level of vacuum in the vacuum line from the venturi manifold can be transmitted to the flow control valve by electronic or fluidic carriers.

Those skilled in the art will readily recognize numerous adaptations and modifications which can be made to the venturi-based compensated vacuum system of the present invention which will result in an improved regulation of a vacuum source yet all of which will fall within the scope and spirit of the present invention as defined in the following claims. Accordingly, the invention is to be limited only by the following claims and their equivalents.

## Claims

1. A vacuum system for providing a regulated source of a vacuum, said system comprising a venturi manifold having a venturi formed therein, said manifold having a source gas inlet for connection to a source gas and to direct that source gas to enter the venturi and a bleed gas outlet for allowing the source gas to leave the venturi manifold after passing through the venturi, a flow control valve for controlling the flow of source gas to the source gas inlet, said venturi manifold having a vacuum supply outlet communicating with the venturi to provide a source of vacuum from the venturi manifold, means for determining the level of vacuum from the vacuum supply outlet and a feedback system to change the flow of source gas entering the venturi depending upon the level of vacuum sensed from the vacuum supply outlet.

2. The vacuum system as defined in claim 1 wherein the feedback is configured to control the flow control valve to change the flow of source gas entering the venturi.

3. The vacuum system as defined in claim 1 wherein said system includes a vacuum conduit in communication with said vacuum supply outlet and said means for determining the level of vacuum from the vacuum supply outlet comprises a means for determining the level of vacuum in said vacuum conduit.

4. A medical vacuum system for supplying vacuum to a patient by means of a vacuum utilization device, said medical vacuum system comprising a venturi manifold having a venturi formed therein, said manifold having a source gas inlet for connection to a source of pressurized gas and to direct that source gas to enter the venturi and a bleed gas outlet for allowing the source gas to leave the venturi manifold after passing through the venturi, a flow control valve for controlling the flow of source gas passing through the venturi, said venturi manifold having a vacuum supply outlet communicating with the venturi to provide a source of vacuum from the venturi manifold, a vacuum conduit for communicating between the vacuum supply outlet and the vacuum utilization device for administering the vacuum to a patient, means for determining the level of vacuum in the vacuum conduit and a feedback system to set the flow of source gas entering the venturi depending upon the level of vacuum sensed in the vacuum conduit from the vacuum supply outlet to a patient.

5. The vacuum system as defined in claim 1 or claim 4 wherein the vacuum utilization device is a medial patient suction system, e.g. a resuscitator.

6. The vacuum system as defined in claim 1 or claim 4 wherein the feedback system comprises a variable volume chamber having an actuator that moves in accordance with the change of volume in the variable volume chamber and wherein the change in volume is related to the level of vacuum from the vacuum supply outlet and wherein said actuator controls the setting of the flow control valve.

7. The vacuum system as defined in claim 6 wherein said variable volume chamber comprises a chamber having a movable wall or a chamber having a flexible diaphragm.

8. The medical vacuum system as defined in claim 4 wherein said means for determining the level of vacuum in the vacuum conduit comprises an electronic vacuum sensor and wherein said flow control valve is operable by an electrical signal from said electronic vacuum sensor.

9. A method of providing a regulated source of vacuum, said method comprising the steps of:
providing a venturi manifold having a supply gas inlet, a supply gas outlet and a venturi formed therebetween having a throat, the venturi manifold further having a vacuum outlet,
supplying a source of pressurized supply gas to the supply gas inlet and passing the supply gas through the venturi to be discharged through the supply gas outlet, thereby forming a vacuum at the throat of the venturi,
communicating the vacuum formed at the venturi throat to the vacuum outlet,
providing a valve to control the flow of supply gas to the supply gas inlet,
sensing the level of vacuum at or proximate to the vacuum outlet,
adjusting the valve to control the flow of supply gas to the supply gas inlet in accordance with the level of vacuum sensed at or proximate to the vacuum outlet.

10. The method as defined in claim 9 wherein the step of adjusting the valve comprises providing a vacuum regulator and communicating the vacuum regulator with the level of vacuum at or proximate to the vacuum outlet to adjust the valve.

11. The method as defined in claim 9 wherein the step of sensing the level of vacuum at or proximate to the vacuum outlet comprises providing an electronic vacuum sensor and where the step of adjusting the valve comprises transmitting an electrical signal from said electronic sensor indicative of the level of vacuum at or proximate to said vacuum outlet to adjust the valve.
